# EUROPÄISCHE PATENTSCHRIFT

(11) **EP 3 386 559 B1**
(45) Veröffentlichungstag und Bekanntmachung des Hinweises auf die Patenterteilung: **23.03.2022**
(21) Anmeldenummer: 16808998.5
(22) Anmeldetag: 07.12.2016
(51) Int. Cl.: A61M 1/00

(54) **ANSCHLUSSVORRICHTUNG ZUR VERWENDUNG BEI DER UNTERDRUCKTHERAPIE VON WUNDEN**
CONNECTION DEVICE FOR USE IN THE NEGATIVE PRESSURE THERAPY OF WOUNDS
DISPOSITIF DE RACCORDEMENT DESTINÉ À ÊTRE UTILISÉ DANS LE TRAITEMENT DE PLAIES PAR PRESSION NÉGATIVE

(30) Priorität: 09.12.2015 DE 102015224746
(43) Veröffentlichungstag der Anmeldung: 17.10.2018
(73) Patentinhaber: Paul Hartmann AG, 89522 Heidenheim (DE)
(72) Erfinder: CROIZAT, Pierre, 89542 Herbrechtingen (DE); ECKSTEIN, Axel, 89522 Heidenheim (DE); HOFSTETTER, Juergen, 52349 Düren (DE); LOEFFLER, Swen, 96465 Neustadt/Coburg (DE)
(74) Vertreter: DREISS Patentanwälte PartG mbB
(86) Internationale Anmeldenummer: PCT/EP2016/080073
(87) Internationale Veröffentlichungsnummer: WO 2017/097834

(56) Entgegenhaltungen:
- WO-A1-2014/066057
- US-A1- 2011 178 481

## Beschreibung

Die Erfindung betrifft eine Anschlussvorrichtung zur Verwendung bei der Unterdrucktherapie von Wunden, mit einem mit Unterdruck und fluiden Medien beaufschlagbaren biegsamen Leitungsmittel mit wenigstens zwei Lumen und mit einem mit dem Leitungsmittel unlösbar verbundenen Anschlusskörper, wobei wenigstens ein Lumen als Sauglumen zum Beaufschlagen mit Unterdruck und wenigstens ein Lumen als Zuführlumen zum Zuführen eines fluiden Mediums vorgesehen ist, wobei der Anschlusskörper aus einem elastomeren biegsamen Material ausgebildet und auf einen die Wunde überfangenden und gegen die Atmosphäre dichtend abschließenden Unterdruckverband aufbringbar ist, wobei das Leitungsmittel im Betrieb der Anschlussvorrichtung durch den Anschlusskörper hindurch und durch wenigstens eine Öffnung in dem Unterdruckverband hindurch mit dem Wundraum kommuniziert, wobei der Anschlusskörper einen zentral angeordneten sich emporerhebenden ersten Bereich, welcher das Leitungsmittel aufnimmt, und einen sich in einer Umfangsrichtung um den ersten Bereich durchgehend herum erstreckenden und demgegenüber flacher bauenden krempenförmigen zweiten Bereich aufweist, der eine Anlageebene bildet und mittels dessen der Anschlusskörper flächenhaft gegen eine wundabgewandte Oberseite des Unterdruckverbands direkt oder unter Zwischenordnung von Haftvermittlern anlegbar und dichtend anfügbar ist, wobei sich an ein freies Ende des in dem zentralen ersten Bereich mündenden Zuführlumens des Leitungsmittels ein allseits abgeschlossener von dem zentralen ersten Bereich gebildeter Leitungsabschnitt anschließt, der erst an einem distalen Ende in einen Innenraum des zentralen ersten Bereichs mündet und von dort aus in Strömungskommunikation mit dem Sauglumen des Leitungsmittels steht.

In der jüngeren Vergangenheit hat die Unterdruckbehandlung von Wunden, insbesondere von tiefen und daher a priori problematisch heilenden Wunden, eine zunehmende Bedeutung erlangt. Dabei bedeutet Unterdruckbehandlung, dass ein an sich der umgebenden Atmosphäre ausgesetzter Körper- oder Wundbereich durch einen Verband druckdicht bzw. unterdruckdicht gegen die Umgebung, also die Atmosphäre, in der wir leben und atmen, abgeschlossen wird, wobei innerhalb des abgeschlossenen Wundbereichs ein gegenüber dem Atmosphärendruck verringerter Druck, mithin also Unterdruck relativ zur Atmosphäre angelegt und dauerhaft oder intermittierend aufrechterhalten wird. Wenn auf dem hier in Rede stehenden Gebiet von Unterdruck die Rede ist, so wird hiermit ein Druckbereich verstanden, der typischerweise zwischen 0 und 250 mmHg (mm Quecksilbersäule) unterhalb des umgebenden Atmosphärendrucks liegt. Es hat sich gezeigt, dass dies der Wundheilung förderlich ist. Für den unterdruckdichten Abschluss der Wunde wird ein Unterdruckverband vorgesehen, der beispielsweise eine druck- bzw. unterdruckdichte Folienschicht umfassen kann, die typischerweise mit einem die Wunde umgebenden unversehrten Körperbereich verklebt ist, so dass auf diese Weise ein dichter Abschluss erreicht werden kann. Um ausgehend von einer Unterdruck erzeugenden Einrichtung, also einer Unterdruckpumpe im weitesten Sinn, einen Unterdruck an den Wundraum heranzuführen und dort aufrechtzuerhalten, können bei hier in Rede stehenden Systemen zur Unterdrucktherapie von Wunden mit Unterdruck beaufschlagbare Leitungsmittel verwendet werden, die mittels einer Anschlussvorrichtung mit dem Unterdruckverband zusammenwirken, um Unterdruck an bzw. in den Wundraum zu bringen.

Eine Anschlussvorrichtung der eingangs genannten Art ist bekannt aus DE 10 2012 214 178 A1 und aus US 2011/0178481 A1. Eine ähnliche Anschlussvorrichtung, jedoch mit einem Prüflumen zur Druckmessung anstelle eines Zuführlumens, ist bekannt aus WO 2014/066057 A1. Dessen ungeachtet sind Anschlussvorrichtungen in den vergangenen Jahren in großem Umfang vorgeschlagen worden. Bei der Gestaltung solcher Anschlussvorrichtungen besteht ein gewisser Zielkonflikt dahingehend, die Anschlussvorrichtung einerseits aus einem biegsamen nachgiebigen Material zu fertigen, um einen hohen Tragkomfort bereitzustellen, jedoch andererseits unter allen Betriebsbedingungen und Belastungen sicherzustellen, dass die Anschlussvorrichtung und deren unterdruck- oder medienführende Lumen nicht derart zusammengedrückt werden, dass eine Kommunikation, d. h. eine Zuführung und Abführung von fluiden Medien, nicht mehr bestimmungsgemäß funktioniert. Diesbezüglich wurden auch Ausführungsformen vorgeschlagen, bei denen die Anschlussvorrichtung dreidimensional über der Anlageebene aufbaut, was die Gefahr des Abdrückens von Lumen und damit der Beeinträchtigung der Funktionsfähigkeit der Anschlussvorrichtung stark reduziert, da verstärkende Wandungen vorgesehen werden können. Auf der anderen Seite ist hiermit eine erhebliche Reduzierung des Tragkomforts verbunden. Bei anderen sehr flachbauenden Anschlussvorrichtungen musste ein Innenraum der Anschlussvorrichtung räumlich sehr begrenzt ausgebildet werden, oder es mussten im Innenraum zahlreiche Abstützelemente in Form von Vorsprüngen und Rippen vorgesehen werden, die sich zwischen einer wundzugewandten und einer wundabgewandten Wandung der Anschlussvorrichtung erstreckten. Demgemäß sind mehrere, jedoch verhältnismäßig kleine Öffnungen in der wundzugewandten Wandung des Anschlusskörpers vorgesehen, um mit dem Wundraum zu kommunizieren.

Der vorliegenden Erfindung liegt die Aufgabe zugrunde, eine Anschlussvorrichtung der eingangs genannten Art dahingehend weiter zu verbessern, dass eine Unterdruckkommunikation auch unter erschwerten Bedingungen sicher gewährleistet ist, wobei ferner die Verstopfungsgefahr der Eintrittsöffnungen oder des Innenraums der Anschlussvorrichtung nicht erhöht, sondern eher reduziert wird.

Diese Aufgabe wird bei einer Anschlussvorrichtung der eingangs genannten Art erfindungsgemäß dadurch gelöst, dass der zentrale erste Bereich eine einzige zum Unterdruckverband hin in der Anlageebene mündende und zusammenhängende Öffnung aufweist, welche den Innenraum des zentralen ersten Bereichs mit der wenigstens einen Öffnung in dem Unterdruckverband verbindet, so dass durch diese zusammenhängende Öffnung hindurch das wenigstens eine Sauglumen mit dem Wundraum kommuniziert, und dass in diese zusammenhängende Öffnung mit dem ersten Bereich einstückige und in der Anlageebene erstreckte und frei endende fingerförmige Fortsätze einragen, die eine Eindringverhinderung für aus dem Wundraum angesaugte Verband- oder Wundeinlagematerialien bilden.

Vorzugsweise sind der Anschlusskörper einschließlich der damit einstückig ausgebildeten in die zusammenhängende Öffnung einragenden Fortsätze und vorzugsweise auch das Leitungsmittel aus PVC gebildet. PVC stellte sich bei Untersuchungen der Anmelderin als vorteilhafter Kompromiss zwischen Eigenstabilität und als angenehm empfundener Nachgiebigkeit und Verformbarkeit des Materials dar.

Mit der vorliegenden Erfindung wurde festgestellt, dass insbesondere beim Ausführen eines Spülvorgangs, wenn also fluide Spülmedien über einen Instillationsanschluss zu dem Anschlusskörper geleitet und von dort wieder abgesaugt werden, oder wenn große Mengen an Wundexsudat in den Anschlusskörper eingesogen werden, eine erhebliche Gefahr besteht, dass Verband- oder Wundeinlagematerialien mit angesaugt werden und im Inneren des Anschlusskörpers Verstopfungen bewirken. Diese Gefahr wird noch erhöht durch Abstützungen innerhalb des Innenraums des Anschlusskörpers. Um die Gefahr des Eindringens solcher Materialien in den Anschlusskörper zu verringern, wurden seither eher kleine Öffnungen in der Anlageebene des Anschlusskörpers vorgesehen. Hierdurch wird aber wiederum der Strömungsquerschnitt reduziert. Mit der vorliegenden Erfindung wird demgegenüber vorgeschlagen, eine einzige und dementsprechend flächenmäßig ausladendere und in der Anlageebene in sich zusammenhängende Öffnung vorzusehen, die in viel geringerem Maße dazu neigt, abgedrückt oder verschlossen zu werden. Um dem Problem von in den Anschlusskörper eindringenden Materialien aus dem Wundraum zu begegnen, wird weiter vorgeschlagen, dass in diese zusammenhängende Öffnung Fortsätze einragen, die eine Eindringverhinderung für aus dem Wundraum angesaugte Verband- oder Wundeinlagematerialien bilden. Diese Fortsätze sind einstückig mit dem biegsamen Material des Anschlusskörpers ausgebildet, sie bauen flach und sind in Betrachtung orthogonal zur Anlageebene insbesondere im Wesentlichen parallel zueinander ausgebildet. Obschon durch diese Fortsätze die lichte Querschnittsfläche der Öffnung wiederum etwas reduziert wird, steht insgesamt dennoch eine große Durchtrittsfläche zur Unterdruck- und Medienkommunikation zwischen dem Wundraum und dem Innenraum des Anschlusskörpers zur Verfügung, wobei durch die in diese Öffnung einragenden Fortsätze das genannte Verstopfungsproblem durch eindringende Materialien wesentlich reduziert werden kann.

In weiterer Ausbildung der Erfindung erweist es sich als vorteilhaft, wenn die Fortsätze sich bis höchstens 80 %, insbesondere bis höchstens 70 %, insbesondere bis höchstens 60 % und weiter insbesondere bis höchstens 50 % der Abmessung der Öffnung in Richtung des jeweiligen Fortsatzes erstrecken. Die ihrerseits vorzugsweise fingerförmigen oder jedenfalls einer erkennbaren Richtung folgenden Fortsätze haben somit einen Abstand von der in dieser Richtung liegenden Begrenzung der Öffnung. Zwischen dem freien Ende des jeweiligen Vorsprungs und der Öffnungsbegrenzung ist also eine ausladende lichte Durchtrittsfläche, die eine gute Unterdruck- und Medienkommunikation erlaubt.

Es erweist sich weiter als vorteilhaft, wenn Fortsätze nach Art eines Kamms, also von einer Seite der Öffnungsbegrenzung ausgehend und insbesondere parallel zueinander erstreckt sind und dabei - wie vorstehend erwähnt - von der gegenüberliegenden Öffnungsbegrenzung beanstandet sind, sodass dort ein zusammenhängender nicht durch Fortsätze begrenzter Bereich gebildet ist.

Es erweist sich weiter als vorteilhaft, wenn die Fläche der Fortsätze in der Anlageebene höchstens 60 %, insbesondere höchstens 50 %, insbesondere höchstens 40 % und weiter insbesondere höchstens 35 % der von der zusammenhängenden Öffnung umschlossenen Fläche einschließlich der Fläche der Fortsätze aufweist.

Die Dicke der Fortsätze orthogonal zu der Anlageebene betrachtet kann in vorteilhafter Weise 1 bis 3 mm betragen.

Es erweist sich weiter als vorteilhaft, dass die Fortsätze bezüglich der Anlageebene elastisch biegsam auslenkbar sind. Sie lassen sich also quer oder orthogonal zu dieser Anlageebene auslenken, und zwar in Richtung auf die Wunde, beispielsweise bei Zuführung von Instillationsfluid in Richtung auf die Wunde, oder in den Innenraum des Anschlusskörpers hinein, etwa infolge eines starken Volumenstroms im Anschluss an einen Spül- oder Instillationsvorgang oder zu Beginn einer Unterdruckbehandlung, wenn der Unterdruck bis zum Wundraum hin erst aufgebaut wird.

Nach einem weiteren Erfindungsgedanken erweist es sich als vorteilhaft, wenn die Auslenkung der Fortsätze durch Anlage der Fortsätze an eine Innenseite einer wundabgewandten Wandung des ersten zentralen Bereichs begrenzt ist. Hierfür erweist es sich als vorteilhaft, wenn die Innenseite der wundabgewandten Wandung eine in Richtung auf die Wunde in den Innenraum hineinerstreckte Rippe oder wenigstens einen Vorsprung aufweist, gegen welche die Fortsätze anlegbar sind. Auf diese Weise ist es ausreichend, wenn in dem Innenraum des zentralen ersten Bereichs des Anschlusskörpers in Projektion lediglich oberhalb der Fortsätze ein Vorsprung vorgesehen ist, um den Innenraum zu stabilisieren, für den Fall, dass von außen Druck auf den Anschlusskörper ausgeübt wird.

Es wird auch vorgeschlagen, den Anschlusskörper und die in der Anlageebene mündende zusammenhängende Öffnung so auszubilden, dass diese Öffnung eine lichte Durchtrittsfläche (also ohne die Fläche der Fortsätze) von 30 % bis 60 % der auf die Anlageebene projizierten Fläche des zentralen ersten Bereichs aufweist.

Im Hinblick auf eine flache Bauform erweist es sich als vorteilhaft, wenn der zentrale erste Bereich ungefähr quaderförmig, vorzugsweise mit verrundeten Ecken und Kanten, ausgebildet ist, wobei sich die kürzeste Kante der Quaderform orthogonal zu dem krempenförmigen zweiten Bereich erstreckt. Ihre Länge beträgt vorzugsweise höchstens 7 mm, wobei die Länge und Breite der anderen Kanten vorzugsweise zwischen 20 mm und 50 mm, insbesondere zwischen 20 mm und 40 mm liegen.

Weiter erweist es sich als vorteilhaft, wenn der Anschlusskörper aus einem einzigen den zentralen ersten Bereich und den krempenförmigen zweiten Bereich bildenden einstückig ausgebildeten Spritzgießteil besteht. Der gesamte Anschlusskörper ist also aus ein und demselben spritzgegossenen elastisch nachgiebigen Material, vorzugsweise PVC, ausgebildet. Er kann jedoch zusätzliche Klebeschichten oder noch näher zu beschreibende Applikationssysteme zum unterdruckdichten Anhaften an die wundabgewandte Außenseite eines Unterdruckverbands aufweisen.

Weiter erweist es sich als vorteilhaft, wenn der erste und/oder zweite Bereich transparent ausgebildet ist. Der zweite krempenförmige Bereich weist vorzugsweise eine Dicke orthogonal zu der Anlageebene von 0,5 bis 1,5 mm auf.

Der zentrale erste Bereich weist eine Höhe oberhalb des krempenförmigen zweiten Bereichs in Richtung orthogonal zu der Anlageebene von höchstens 12 mm, insbesondere von höchstens 7 mm, bei einer größten Abmessung in der Anlageebene von höchstens 50 mm, insbesondere von höchstens 35 mm auf.

Nach einem weiteren Erfindungsgedanken erweist es sich als besonders vorteilhaft, dass das Leitungsmittel außerhalb des zentralen ersten Bereichs nicht mit dem krempenförmigen zweiten Bereich gefügt ist, so dass das Leitungsmittel aufgrund seiner Biegsamkeit gelenkig gegenüber dem zentralen ersten Bereich auslenkbar ist, ohne dass hierdurch wesentliche Kräfte auf den krempenförmigen zweiten Bereich übertragen werden, welche zu einer Beeinträchtigung der Fügeverbindung zum Unterdruckverband hin führen könnten.

Wie bereits angedeutet, erweist es sich als vorteilhaft, wenn der Anschlusskörper herstellerseitig bereits ein Applikationssystem erhält. Hierfür erweist es sich als vorteilhaft, wenn der Anschlusskörper auf der wundabgewandten oder auf der wundzugewandten Seite des krempenförmigen zweiten Bereichs einen Befestigungsfilm unlösbar trägt, der nach radial außen über einen Umfangsrand des krempenförmigen zweiten Bereichs um wenigstens 5 mm, insbesondere um wenigstens 10 mm vorsteht, und auf seiner wundzugewandten Seite eine Kleberschicht aufweist, mittels derer er auf den Unterdruckverband haftend aufbringbar ist. Wenn der Befestigungsfilm auf der wundabgewandten Seite des krempenförmigen zweiten Bereichs appliziert ist, so haftet er mit seiner Kleberschicht auch auf diesem krempenförmigen zweiten Bereich.

Hierbei erweist es sich als vorteilhaft, wenn der Befestigungsfilm auf der wundzugewandten Seite wenigstens einen ablösbaren Schutzfolienabschnitt und auf der wundabgewandten Seite wenigstens einen Stützfolienabschnitt aufweist, der nach der Aufbringung der Anschlussvorrichtung auf den Unterdruckverband ablösbar ist.

Gegenstand der Erfindung ist auch ein Verfahren zum Herstellen einer Anschlussvorrichtung mit den Merkmalen des Anspruchs 19.

Des Weiteren wird Schutz beansprucht für ein System zur Unterdrucktherapie von Wunden mit den Komponenten und Merkmalen des Anspruchs 20.

Weitere Merkmale, Einzelheiten und Vorteile der Erfindung ergeben sich aus den beigefügten Patentansprüchen und der zeichnerischen Darstellung und nachfolgenden Beschreibung einer bevorzugten Ausführungsform der Erfindung.

In der Zeichnung zeigt:
Figur 1a, b perspektivische Ansichten einer erfindungsgemäßen Anschlussvorrichtung mit einem Applikationssystem;
Figur 2a eine Draufsicht auf die Anschlussvorrichtung nach Figuren 1a, b ohne Applikationssystem;
Figur 2b eine Ansicht der Anschlussvorrichtung nach Figur 2a von unten mit Blick auf eine wundzugewandte Anlageebene;
Figur 2c eine vergrößerte Ansicht der Figur 2b;
Figuren 3a, b eine Schnittansicht des Leitungsmittels sowie eine Draufsicht auf das Leitungsmittel der erfindungsgemäßen Anschlussvorrichtung;
Figuren 4a,b perspektivische Ansichten eines Anschlusskörpers der erfindungsgemäßen Anschlussvorrichtung;
Figur 4c eine Ansicht des Anschlusskörpers mit Blick auf eine Einstecköffnung für das Leitungsmittel;
Figuren 4d,e Schnittansichten durch den Anschlusskörper mit Schnittebenen A-A bzw. B-B in Figur 2b und 4c;
Figur 5 eine Schnittansichten durch den Anschlusskörper entsprechend Figur 4e jedoch mit Applikationssystem.

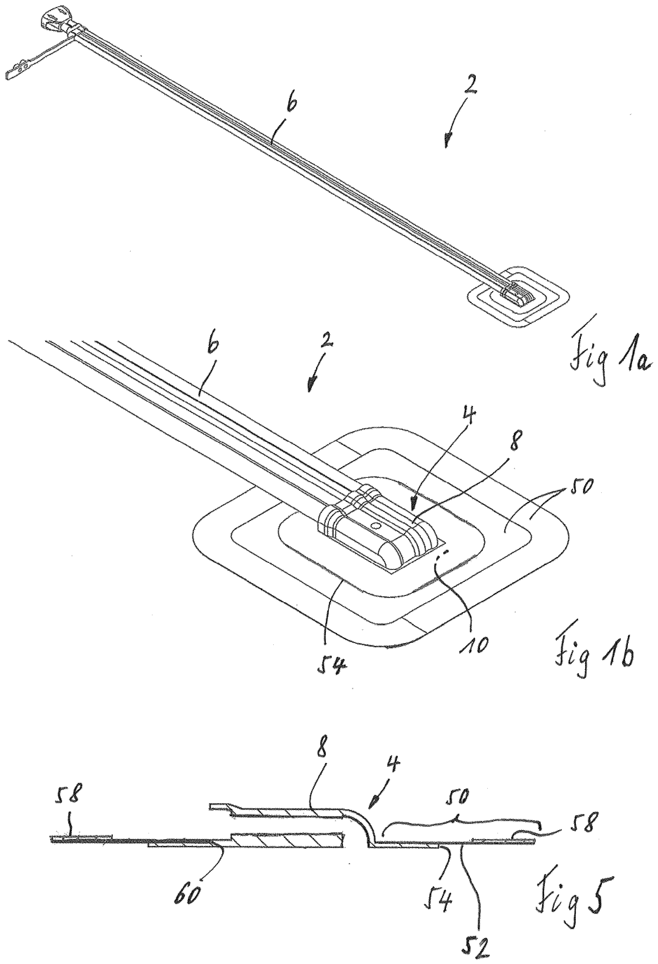

Die Figuren zeigen eine insgesamt mit dem Bezugszeichen 2 bezeichnete erfindungsgemäße Anschlussvorrichtung zur Verwendung bei der Unterdrucktherapie von Wunden. Die Anschlussvorrichtung 2 umfasst einen Anschlusskörper 4, der auf einen nicht dargestellten die Wunde überfangenden und gegen die Atmosphäre dichtend abschließenden Unterdruckverband vorzugsweise unlösbar haftend aufbringbar ist, und ein unlösbar mit dem Anschlusskörper 4 verbundenes Leitungsmittel 6. Über das Leitungsmittel 6 wird eine Unterdruck- oder Strömungskommunikation zwischen dem Inneren des Anschlusskörpers 4 und einer Unterdruck erzeugenden Einrichtung oder einem weiteren dorthin führenden Leitungsmittel hergestellt. Die Unterdruck- oder Strömungskommunikation zur Wunde erfolgt durch eine noch zu beschreibende Öffnung in dem Anschlusskörper und eine Öffnung in dem die Wunde überfangenden Unterdruckverband. Die in Figuren 1a, b dargestellte Anschlussvorrichtung 2 weist außerdem ein auf der wundabgewandten Seite des Anschlusskörpers 4 angefügtes Applikationssystem 50 auf, das in Zusammenhang mit Figur 5 beschrieben wird und nachfolgend zunächst weggelassen ist.

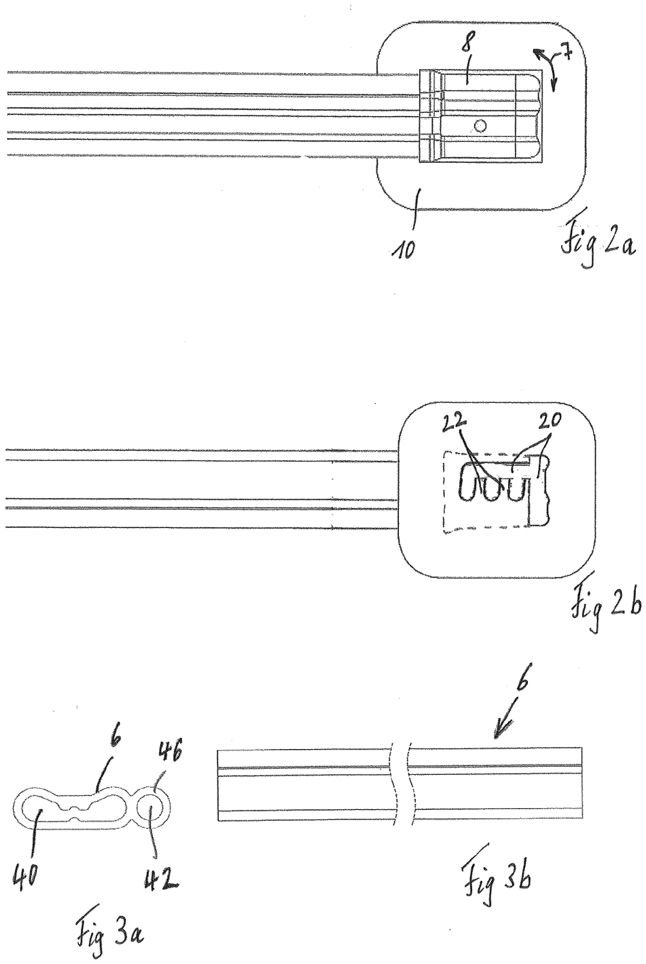

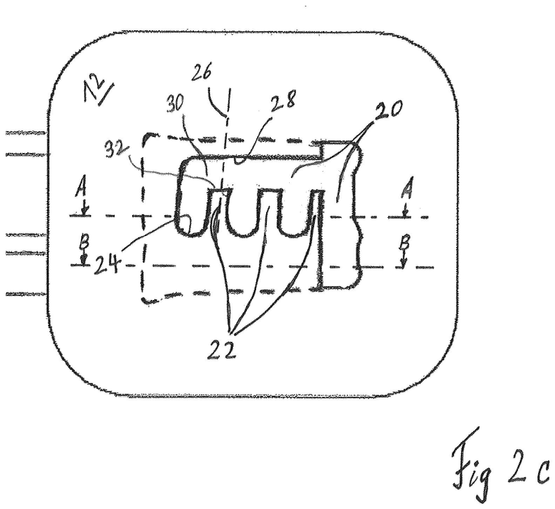

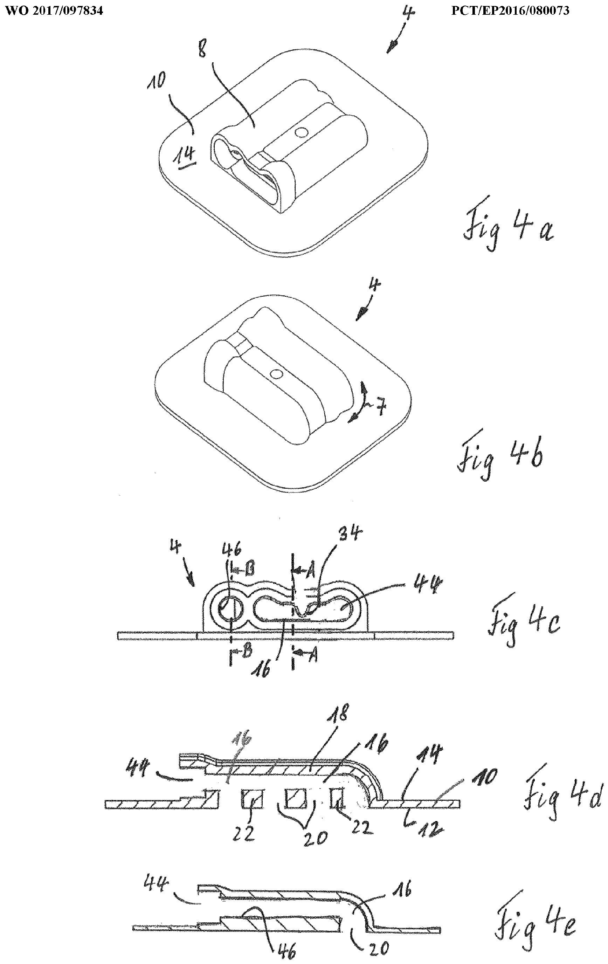

Der Anschlusskörper 4 umfasst einen zentralen ersten Bereich 8, welcher auf noch zu beschreibende Weise das Leitungsmittel 6 aufnimmt, und einen sich in einer Umfangsrichtung 7 um den ersten Bereich 8 durchgehend herum erstreckenden und demgegenüber flacher bauenden krempenförmigen zweiten Bereich 10. Der krempenförmige zweite Bereich 10 bildet eine Anlageebene 12 (Figur 2b), mit der der Anschlusskörper 4 flächenhaft gegen die wundabgewandte Außenseite des Unterdruckverbands anlegbar und fixierbar ist. Der zentrale erste Bereich 8 und der krempenförmigen zweite Bereich 10 sind einstückig aus demselben Material als ein Spritzgießteil hergestellt. Der zentrale erste Bereich 8 erhebt sich wenige Millimeter von der wundabgewandten Seite 14 des krempenförmigen zweiten Bereichs 10. Er ist beispielhaft ungefähr quaderförmig aber gleichwohl sehr flach ausgebildet. Er umfasst einen Innenraum 16 (Figur 4), der nach oben hin von einer wundabgewandten Wandung 18 begrenzt ist und zur Wunde hin über eine einzige in der Anlageebene 12 zusammenhängende ausladende Öffnung 20 in Richtung auf die Wunde bzw. die Öffnung in dem die Wunde überfangenden Unterdruckverband ausmündet. In diese zusammenhängende Öffnung 20 des Anschlusskörpers 4 ragen mehrere fingerförmige Fortsätze 22 ein. In dem beispielhaft und bevorzugt dargestellten Fall erstrecken sich die fingerförmigen Fortsätze 22 ausgehend von einer die Öffnung 20 begrenzenden Seite 24 ungefähr parallel zueinander. Die Fortsätze 22 haben dabei eine Erstreckungsrichtung 26, wie in Figur 2c angedeutet ist. In dieser Richtung 26 sind sie beabstandet von der gegenüberliegenden Seite 28 der Öffnung 20. Daher ragen die fingerförmigen Fortsätze 22 zwar in die Öffnung 20 ein und enden dort frei; dennoch ist eine große lichte Durchtrittsfläche im Bereich 30 zwischen einem jeweiligen freien Ende 32 der Vorsprünge 22 und der gegenüberliegenden Seite 28 oder Öffnungsbegrenzung der Öffnung 20 gebildet. Die fingerförmigen Fortsätze 22 sind elastisch nachgiebig auslenkbar bezüglich der Anlageebene 12 des Anschlusskörpers 4. Sie bilden eine Eindringverhinderung für Verband-oder Wundeinlagematerialien, etwa aus Schaum, Mull oder dergleichen.

Wenn die fingerförmigen Fortsätze 22 in den Innenraum 16 des zentralen ersten Bereichs 8 ausgelenkt werden, so sind sie an einen von der wundabgewandten Wandung 18 in Richtung auf die Wunde vorstehenden Vorsprung 34 anlegbar, wobei dieser Vorsprung 34 somit die Auslenkbarkeit der fingerförmigen Fortsätze 22 begrenzt. Dieser Vorsprung 34 ist insbesondere in Form einer einzigen in einer Längsrichtung des Leitungsmittels 6 erstreckten Rippe ausgebildet.

Der Innenraum 16 des zentralen ersten Bereichs 8 kommuniziert einerseits über die beschriebene Öffnung 20 mit dem Wundraum und andererseits mit wenigstens einem Sauglumen 40 und wenigstens einem Zuführlumen 42 des Leitungsmittels 6 (Figur 3a). Für den herstellerseitigen Anschluss des Leitungsmittels 6 an den zentralen ersten Bereich 8 ist dort eine parallel zur Anlageebene 12 ausmündende Einstecköffnung 44 ausgebildet, die der Außenkontur des Leitungsmittels 6 entspricht. Das Leitungsmittel 6 ist mit seinem freien Ende in die Einstecköffnung 44 einsteckbar und dort stoffschlüssig und für den bestimmungsgemäßen Gebrauch vorzugsweise unlösbar und unterdruckdicht fixiert. Dabei mündet das Sauglumen 40 in den Innenraum 16 des zentralen ersten Bereichs 8. An ein freies Ende des Zuführlumens 42 des Leitungsmittels 6 schließt sich hingegen ein allseits umschlossener von dem zentralen ersten Bereich 8 gebildeter Leitungsabschnitt 46 an, der sich in der Längsrichtung durch den zentralen ersten Bereich 8 erstreckt und erst am gegenüberliegenden Ende in den Innenraum 16 mündet. Es erweist sich als vorteilhaft, dass im Bereich des distalen Endes dieses Leitungsabschnitts 46 auch die zusammenhängende Öffnung 20 ausgebildet ist, sodass dort zugeführte Instillationsflüssigkeit in den Wundraum gelangen kann. Wenn durch das Zuführlumen 42 Spülflüssigkeit in den Innenraum 16 zugeführt wird, so erweist es sich als vorteilhaft, dass die Spülflüssigkeit auf der gegenüberliegenden Seite der Einstecköffnung 44 für das Leitungsmittel 6 in den Innenraum 16 eingeleitet wird.

Das eingangs schon erwähnte Applikationssystem 50 umfasst einen dünnen Befestigungsfilm 52, der nach radial außen über einen Umfangsrand 54 des Anschlusskörpers 4 vorsteht und auf seiner wundzugewandten Seite eine nicht dargestellte Kleberschicht trägt, die von ablösbaren einander überlappenden Schutzfolienabschnitten (nicht dargestellt) überfangen ist. Ferner ist auf der wundabgewandten Seite ein formstabilisierender Stützfolienabschnitt 58 vorgesehen, der nach der Aufbringung der Anschlussvorrichtung 2 auf den Unterdruckverband abgelöst wird. Der Befestigungsfilm 52 ist transparent ausgebildet, weshalb in Figur 1b die Kontur und der Umfangsrand 54 des krempenförmigen zweiten Bereichs 10 ersichtlich ist.

Der Befestigungsfilm 52 weist vorzugsweise eine Öffnung 60 auf, mit der er über den zentralen ersten Bereich 8 stülpbar solange das Leitungsmittel 6 noch nicht in den Anschlusskörper 4 eingefügt ist, und auf die wundabgewandte Seite 14 des krempenförmigen zweiten Bereichs 10 haftend aufbringbar ist. Im Anschluss daran wird das Leitungsmittel 10 in die Einstecköffnung 44 eingesteckt und dichtend fixiert.

## Patentansprüche

1. Anschlussvorrichtung (2) zur Verwendung bei der Unterdrucktherapie von Wunden, mit einem mit Unterdruck und fluiden Medien beaufschlagbaren biegsamen Leitungsmittel (6) mit wenigstens zwei Lumen und mit einem mit dem Leitungsmittel (6) unlösbar verbundenen Anschlusskörper (4), wobei wenigstens ein Lumen als Sauglumen (40) zum Beaufschlagen mit Unterdruck und wenigstens ein Lumen als Zuführlumen (42) zum Zuführen eines fluiden Mediums vorgesehen ist, wobei der Anschlusskörper (4) aus einem elastomeren biegsamen Material ausgebildet und auf einen die Wunde überfangenden und gegen die Atmosphäre dichtend abschließenden Unterdruckverband aufbringbar ist, wobei das Leitungsmittel (6) im Betrieb der Anschlussvorrichtung durch den Anschlusskörper (4) hindurch und durch wenigstens eine Öffnung in dem Unterdruckverband hindurch mit dem Wundraum kommuniziert, wobei der Anschlusskörper (4) einen zentral angeordneten sich emporerhebenden ersten Bereich (8), welcher das Leitungsmittel (6) aufnimmt, und einen sich in einer Umfangsrichtung (7) um den ersten Bereich (8) durchgehend herum erstreckenden und demgegenüber flacher bauenden krempenförmigen zweiten Bereich (10) aufweist, der eine Anlageebene (12) bildet und mittels dessen der Anschlusskörper (4) flächenhaft gegen eine wundabgewandte Oberseite des Unterdruckverbands direkt oder unter Zwischenordnung von Haftvermittlern anlegbar und dichtend anfügbar ist, wobei sich an ein freies Ende des in dem zentralen ersten Bereich mündenden Zuführlumens (42) des Leitungsmittels (6) ein allseits abgeschlossener von dem zentralen ersten Bereich gebildeter Leitungsabschnitt (46) anschließt, der erst an einem distalen Ende in einen Innenraum (16) des zentralen ersten Bereichs (8) mündet und von dort aus in Strömungskommunikation mit dem Sauglumen (40) des Leitungsmittels (6) steht, **dadurch gekennzeichnet, dass** der zentrale erste Bereich (8) eine einzige zum Unterdruckverband hin in der Anlageebene (12) mündende und zusammenhängende Öffnung (20) aufweist, welche den Innenraum (16) des zentralen ersten Bereichs (8) mit der wenigstens einen Öffnung in dem Unterdruckverband verbindet, so dass durch diese zusammenhängende Öffnung (20) hindurch das wenigstens eine Sauglumen (40) mit dem Wundraum kommuniziert, und dass in diese zusammenhängende Öffnung (20) mit dem ersten Bereich (8) einstückige und in der Anlageebene (12) erstreckte und frei endende fingerförmige Fortsätze (22) einragen, die eine Eindringverhinderung für aus dem Wundraum angesaugte Verband- oder Wundeinlagematerialien bilden.

2. Anschlussvorrichtung nach Anspruch 1, **dadurch gekennzeichnet, dass** die Fortsätze (22) in Betrachtung orthogonal zur Anlageebene (12) im Wesentlichen parallel zueinander ausgebildet sind.

3. Anschlussvorrichtung nach Anspruch 1 oder 2, **dadurch gekennzeichnet, dass** die Fortsätze (22) sich bis höchstens 80 %, insbesondere bis höchstens 70 %, insbesondere bis höchstens 60 % und weiter insbesondere bis höchstens 50 % der Abmessung der Öffnung (20) in Richtung (20) des jeweiligen Fortsatzes (22) erstrecken.

4. Anschlussvorrichtung nach einem oder mehreren der vorstehenden Ansprüche, **dadurch gekennzeichnet, dass** die Fortsätze (22) nach Art eines Kamms, also von einer Seite (24) der Öffnung ausgehend und insbesondere parallel zueinander erstreckt sind.

5. Anschlussvorrichtung nach einem oder mehreren der vorstehenden Ansprüche, **dadurch gekennzeichnet, dass** die Fläche der Fortsätze (22) in der Anlageebene (12) höchstens 60 %, insbesondere höchstens 50 %, insbesondere höchstens 40 % und weiter insbesondere höchstens 35 % der von der zusammenhängenden Öffnung (20) umschlossenen Fläche einschließlich der Fläche der Fortsätze aufweist.

6. Anschlussvorrichtung nach einem oder mehreren der vorstehenden Ansprüche, **dadurch gekennzeichnet, dass** die Fortsätze (22) eine Dicke orthogonal zu der Anlageebene (12) von 1 bis 3 mm aufweisen.

7. Anschlussvorrichtung nach einem oder mehreren der vorstehenden Ansprüche, **dadurch gekennzeichnet, dass** die Fortsätze (22) bezüglich der Anlageebene (12) elastisch biegsam auslenkbar sind.

8. Anschlussvorrichtung nach Anspruch 7, **dadurch gekennzeichnet, dass** die Auslenkung der Fortsätze (22) durch Anlage der Fortsätze (22) an eine Innenseite einer wundabgewandten Wandung (18) des ersten zentralen Bereichs begrenzt ist.

9. Anschlussvorrichtung nach Anspruch 8, **dadurch gekennzeichnet, dass** die Innenseite der wundabgewandten Wandung (18) eine in Richtung auf die Wunde in den Innenraum (16) hineinerstreckte Rippe oder wenigstens einen Vorsprung (34) aufweist, gegen welche die Fortsätze (22) anlegbar sind.

10. Anschlussvorrichtung nach einem oder mehreren der vorstehenden Ansprüche, **dadurch gekennzeichnet, dass** der Anschlusskörper (4) aus einem einzigen den zentralen ersten Bereich (8) und den krempenförmigen zweiten Bereich (12) bildenden einstückig ausgebildeten Spritzgießteil und gegebenenfalls Haftvermittlungselementen oder -schichten besteht.

11. Anschlussvorrichtung nach einem oder mehreren der vorstehenden Ansprüche, **dadurch gekennzeichnet, dass** der zentrale erste Bereich (8) eine Höhe oberhalb des krempenförmigen zweiten Bereichs (10) in Richtung orthogonal zu der Anlageebene (12) von höchstens 12 mm, insbesondere von höchstens 7 mm, bei einer größten Abmessung in der Anlageebene (12) von höchstens 50 mm, insbesondere von höchstens 35 mm aufweist.

12. Anschlussvorrichtung nach einem oder mehreren der vorstehenden Ansprüche, **dadurch gekennzeichnet, dass** das Leitungsmittel (6) außerhalb des zentralen ersten Bereichs (8)nicht mit dem krempenförmigen zweiten Bereich (10) gefügt ist, so dass das Leitungsmittel aufgrund seiner Biegsamkeit gelenkig gegenüber dem zentralen ersten Bereich (8) auslenkbar ist, ohne dass hierdurch wesentliche Kräfte auf den krempenförmigen zweiten Bereich (10) übertragen werden.

13. Anschlussvorrichtung nach einem oder mehreren der vorstehenden Ansprüche, **dadurch gekennzeichnet, dass** der Anschlusskörper (4) auf der wundabgewandten oder auf der wundzugewandten Seite (14) des krempenförmigen zweiten Bereichs (10) einen Befestigungsfilm (52) unlösbar trägt, der nach radial außen über einen Umfangsrand (54) des krempenförmigen zweiten Bereichs (10) um wenigstens 5 mm, insbesondere um wenigstens 10 mm vorsteht, und auf seiner wundzugewandten Seite eine Kleberschicht aufweist, mittels derer er auf den Unterdruckverband haftend aufbringbar ist.

14. Anschlussvorrichtung nach Anspruch 13, **dadurch gekennzeichnet, dass** der Befestigungsfilm (52) auf der wundzugewandten Seite wenigstens einen ablösbaren Schutzfolienabschnitt und auf der wundabgewandten Seite wenigstens einen Stützfolienabschnitt (58) aufweist, der nach der Aufbringung der Anschlussvorrichtung auf den Unterdruckverband ablösbar ist.

15. Verfahren zum Herstellen einer Anschlussvorrichtung nach einem oder mehreren der vorstehenden Ansprüche 1-14, **dadurch gekennzeichnet, dass** der Anschlusskörper (4) mit zentralem ersten Bereich (8) und krempenförmigem zweiten Bereich (10) einstückig hergestellt und bereitgestellt wird, und dass auf der wundabgewandten Seite des krempenförmigen zweiten Bereichs (10) ein in der Umfangsrichtung (7) vorzugsweise durchgehender Befestigungsfilm (52) mit einer zentralen Ausnehmung (60) vorgesehen wird, indem der Befestigungsfilm (52) mit seiner zentralen Ausnehmung (60) über den zentralen ersten Bereich (8) gestülpt wird und der Befestigungsfilm (52) dann mittels Kleber auf der wundabgewandten Seite (14) des krempenförmigen zweiten Bereichs (10) zum bestimmungsgemäßen Gebrauch unlösbar anhaftet und nach außen über einen Umfangsrand (54) des krempenförmigen zweiten Bereichs (10) übersteht, und dass im Anschluss daran das Leitungsmittel (6) in eine ungefähr parallel zur Anlageebene (12) ausmündende Öffnung (44) des ersten zentralen Bereichs (8) eingesteckt und stoffschlüssig und unterdruckdicht befestigt wird.

16. System zur Unterdrucktherapie von Wunden, mit einem eine Unterdruck erzeugende Einrichtung umfassenden Gerät mit einem Unterdruckanschluss und mit einem Spül- oder Instillationsanschluss, **gekennzeichnet durch** eine Anschlussvorrichtung (4) nach einem oder mehreren der vorstehenden Ansprüche 1-14, wobei ein Sauglumen (40) des Leitungsmittels (6) mit dem Unterdruckanschluss und das Zuführlumen (42) des Leitungsmittels (6) mit dem Spül- oder Instillationsanschluss strömungsverbunden ist.

## Claims

1. A connection device (2) for use in the negative pressure therapy of wounds, with a flexible conducting means (6) which can be subjected to negative pressure and to fluid media and has at least two lumina, and with a connecting body (4) connected non-releasably to the conducting means (6), wherein at least one lumen is provided as suction lumen (40) for subjecting to negative pressure, and at least one lumen is provided as delivery lumen (42) for delivering a fluid medium, wherein the connecting body (4) is made of an elastomeric flexible material and can be applied to a negative pressure dressing which covers the wound and seals the wound off tightly from the atmosphere, wherein the conducting means (6), during operation of the connection device, communicates with the wound space through the connecting body (4) and through at least one opening in the negative pressure dressing, wherein the connecting body (4) has a centrally arranged and raised first region (8), which receives the conducting means (6), and a flange-shaped second region (10) which extends continuously in a circumferential direction (7) about the first region (8), is flatter compared to the latter and forms a contact plane (12), and by means of which the connecting body (4) can be placed flat against an upper side of the negative pressure dressing facing away from the wound, either directly or with interposition of adhesion promoters, and can be joined sealingly thereto, wherein a conducting portion (46) which is closed on all sides and is formed by the central first region adjoins a free end of the delivery lumen (42) of the conducting means (6) opening into the central first region, which conducting portion (46) opens only at a distal end into an interior (16) of the central first region (8) and from there is in fluidic communication with the suction lumen (40) of the conducting means (6), **characterized in that** the central first region (8) has one single continuous opening (20) which leads toward the negative pressure dressing in the contact plane (12), which opening (20) connects the interior (16) of the central first region (8) to the at least one opening in the negative pressure dressing, such that the at least one suction lumen (40) communicates with the wound space through this continuous opening (20), and **in that** finger-shaped extensions (22) integral with the first region (8) and extending in the contact plane (12) protrude with free ends into this continuous opening (20) and form a means for preventing penetration of dressing materials or wound inlay materials aspirated from the wound space.

2. The connection device as claimed in claim 1, **characterized in that** the extensions (22), viewed orthogonally with respect to the contact plane (12), are substantially parallel to one another.

3. The connection device as claimed in claim 1 or 2, **characterized in that** the extensions (22) extend by at most 80%, in particular by at most 70%, in particular by at most 60% and more particularly by at most 50% of the dimension of the opening (20) in the direction (20) of the respective extension (22).

4. The connection device as claimed in one or more of the preceding claims, **characterized in that** the extensions (22) extend in the manner of a comb, i.e. starting from one side (24) of the opening and in particular parallel to one another.

5. The connection device as claimed in one or more of the preceding claims, **characterized in that** the surface area of the extensions (22) in the contact plane (12) has at most 60%, in particular at most 50%, in particular at most 40% and more particularly at most 35% of the surface area enclosed by the continuous opening (20), including the surface area of the extensions.

6. The connection device as claimed in one or more of the preceding claims, **characterized in that** the extensions (22) have a thickness of 1 to 3 mm orthogonal to the contact plane (12).

7. The connection device as claimed in one or more of the preceding claims, **characterized in that** the extensions (22) can deflect in an elastically flexible manner with respect to the contact plane (12).

8. The connection device as claimed in claim 7, **characterized in that** the deflection of the extensions (22) is limited by the extensions (22) bearing on an inner side of a wall (18), facing away from the wound, of the first central region.

9. The connection device as claimed in claim 8, **characterized in that** the inner side of the wall (18) facing away from the wound has a rib, extending into the interior (16) in the direction of the wound, or at least one projection (34) against which the extensions (22) can be placed.

10. The connection device as claimed in one or more of the preceding claims, **characterized in that** the connecting body (4) is composed of a single integrally produced injection molding, which forms the central first region (8) and the flange-shaped second region (12), and optionally of adhesion- promoting elements or layers.

11. The connection device as claimed in one or more of the preceding claims, **characterized in that** the central first region (8) has a height above the flange-shaped second region (10), in a direction orthogonal to the contact plane (12), of at most 12 mm, in particular of at most 7 mm, with a maximum dimension in the contact plane (12) of at most 50 mm, in particular of at most 35 mm.

12. The connection device as claimed in one or more of the preceding claims, **characterized in that** the conducting means (6) outside the central first region (8) is not joined to the flange-shaped second region (10), such that the conducting means, on account of its flexibility, can be deflected in a swiveled manner relative to the central first region (8), without considerable forces thereby being transmitted to the flange-shaped second region (10) .

13. The connection device as claimed in one or more of the preceding claims, **characterized in that**, on the side of the flange-shaped second region (10) facing away from the wound, or on the side (14) of the flange-shaped second region (10) facing toward the wound, the connecting body (4) non-releasably supports a securing film (52) which protrudes radially outward over a peripheral edge (54) of the flange-shaped second region (10) by at least 5 mm, in particular by at least 10 mm, and, on its side facing toward the wound, it has an adhesive layer by means of which it can be applied adhesively to the negative pressure dressing.

14. A connection device as claimed in claim 13, **characterized in that** the securing film (52) has, on the side facing toward the wound, at least one releasable protective film portion, and, on the side facing away from the wound, it has at least one supporting film portion (58), which is detachable after the connection device has been applied to the negative pressure dressing.

15. A method for producing a connection device as claimed in one or more of claims 1-14, **characterized in that** the connecting body (4) with central first region (8) and with flange-shaped second region (10) is produced and supplied in one piece, and **in that** a securing film (52), extending preferably continuously in the circumferential direction (7) and having a central recess (60), is provided on the side of the flange-shaped second region (10) facing away from the wound, the securing film (52) being pushed with its central recess (60) over the central first region (8), after which the securing film (52) then adheres non-releasably by means of adhesive to the side (14) of the flange-shaped second region (10) facing away from the wound for the intended use and protrudes outward beyond a peripheral edge (54) of the flange-shaped second region (10), and **in that** the conducting means (6) is thereafter inserted into an opening (44) of the first central region (8) opening out approximately parallel to the contact plane (12) and is secured cohesively and in a negative-pressure-tight manner.

16. A system for negative pressure therapy of wounds, with an appliance which comprises a negative pressure generator and which has a negative pressure port and a rinsing or instillation port, **characterized by** a connection device (4) as claimed in one or more of claims 1-14, wherein a suction lumen (40) of the conducting means (6) is fluidically connected to the negative pressure port and the delivery lumen (42) of the conducting means (6) is fluidically connected to the rinsing or instillation port.

## Revendications

1. Dispositif de raccordement (2) destiné à être utilisé dans la thérapie par pression négative de plaies, avec un moyen conduit (6) flexible qui peut être soumis à une pression négative et alimenté en milieux fluides et qui présente au moins deux lumières, ainsi qu'avec un corps de raccordement (4) qui est relié de manière inamovible au moyen conduit (6), dans lequel au moins une lumière est prévue en tant que lumière d'aspiration (40) pour appliquer une pression négative et au moins une lumière est prévue en tant que lumière d'alimentation (42) pour amener un milieu fluide, dans lequel ledit corps de raccordement (4) est fait à partir d'un matériau élastomère flexible et peut être appliqué sur un pansement à pression négative recouvrant la plaie et assurant l'étanchéité de celle-ci contre l'atmosphère, dans lequel, en fonctionnement du dispositif de raccordement, le moyen conduit (6) communique avec la zone de la plaie à travers le corps de raccordement (4) et à travers au moins une ouverture dans le pansement à pression négative, dans lequel le corps de liaison (4) comprend une première zone (8) en relief qui est disposée au centre et qui reçoit le moyen conduit (6) ainsi qu'une deuxième zone (10) en forme de bord qui s'étend de manière continue dans une direction circonférentielle (7) autour de la première zone (8) et est plus plate en comparaison de celle-ci et qui forme un plan d'appui (12) et au moyen de laquelle le corps de raccordement (4) peut être appliqué en nappe et joint à étanchéité, soit directement soit en intercalant des promoteurs d'adhérence, contre une face supérieure du pansement à pression négative, qui montre dans la direction opposée à la plaie, dans lequel une extrémité libre de la lumière d'alimentation (42) du moyen conduit (6), qui débouche dans la première zone centrale, est suivie d'une section de conduit (46) qui est fermée de tout côté et formée par ladite première zone centrale et qui, d'abord, débouche à une extrémité distale dans un espace intérieur (16) de la première zone centrale (8) et est, à partir de là, en communication fluidique avec la lumière d'aspiration (40) du moyen conduit (6), **caractérisé par le fait que** la première zone centrale (8) comprend une seule ouverture (20) qui est continue et débouche vers le pansement à pression négative dans le plan d'appui (12) et qui relie ledit espace intérieur (16) de la première zone centrale (8) à ladite au moins une ouverture dans le pansement à pression négative de sorte que ladite au moins une lumière d'aspiration (40) communique avec la zone de la plaie à travers cette ouverture (20) continue, et que dans cette ouverture (20) continue s'étendent des prolongements (22) en forme de doigts qui sont faits d'un seul tenant avec la première zone (8) et s'étendent dans le plan d'appui (12) et se terminent librement et qui forment un moyen d'empêchement de pénétration pour des matériaux de pansement ou d'insert de plaie qui sont aspirés de la zone de la plaie.

2. Dispositif de raccordement selon la revendication 1, **caractérisé par le fait que** les prolongements (22), vus orthogonalement au plan d'appui (12), sont réalisés de manière à être pour l'essentiel parallèles les uns aux autres.

3. Dispositif de raccordement selon la revendication 1 ou 2, **caractérisé par** le fait les prolongements (22) s'étendent jusqu'à 80 % tout au plus, en particulier jusqu'à 70 % tout au plus, en particulier jusqu'à 60 % tout au plus et en outre en particulier jusqu'à 50 % tout au plus de la dimension de l'ouverture (20) en direction du prolongement (22) respectif.

4. Dispositif de raccordement selon l'une ou plusieurs des revendications précédentes, **caractérisé par le fait que** les prolongements (22) s'étendent à la manière d'un peigne, c'est-à-dire à partir d'un côté (24) de l'ouverture, et en particulier parallèlement les uns aux autres.

5. Dispositif de raccordement selon l'une ou plusieurs des revendications précédentes, **caractérisé par le fait que** la surface des prolongements (22) dans le plan d'appui (12) présente 60 % tout au plus, en particulier 50 % tout au plus, en particulier 40 % tout au plus et en outre en particulier 35 % tout au plus de la surface, y compris la surface des prolongements, qui est entourée par l'ouverture (20) continue.

6. Dispositif de raccordement selon l'une ou plusieurs des revendications précédentes, **caractérisé par le fait que** les prolongements (22) présente une épaisseur orthogonale au plan d'appui (12) qui est comprise entre 1 et 3 mm.

7. Dispositif de raccordement selon l'une ou plusieurs des revendications précédentes, **caractérisé par le fait que** les prolongements (22) peuvent être déviés de manière élastiquement flexible par rapport au plan d'appui (12).

8. Dispositif de raccordement selon la revendication 7, **caractérisé par le fait que** la déviation des prolongements (22) est limité **par le fait que** les prolongements (22) sont en appui contre une face intérieure d'une paroi (18) de la première zone centrale, qui montre dans la direction opposée à la plaie.

9. Dispositif de raccordement selon la revendication 8, **caractérisé par le fait que** la face intérieure de la paroi (18) qui montre dans la direction opposée à la plaie présente une nervure s'étendant dans ledit espace intérieur (16) en direction de la plaie ou au moins une saillie (34) contre lesquelles les prolongements (22) peuvent être appliquées.

10. Dispositif de raccordement selon l'une ou plusieurs des revendications précédentes, **caractérisé par le fait que** le corps de raccordement (4) est constitué d'une seule pièce moulée par injection qui forme la première zone centrale (8) et la deuxième zone (12) en forme de bord et qui est faite d'un seul tenant, ainsi que, le cas échéant, d'éléments ou de couches favorisant l'adhérence.

11. Dispositif de raccordement selon l'une ou plusieurs des revendications précédentes, **caractérisé par le fait que** la première zone centrale (8) présente une hauteur au-dessus de la deuxième zone (10) en forme de bord, dans la direction orthogonale au plan d'appui (12), qui est de 12 mm tout au plus, en particulier de 7 mm tout au plus, avec une dimension maximale dans le plan d'appui (12) de 50 mm tout au plus, en particulier de 35 mm tout au plus.

12. Dispositif de raccordement selon l'une ou plusieurs des revendications précédentes, **caractérisé par le fait que** le moyen conduit (6) n'est pas joint à la deuxième zone (10) en forme de bord à l'extérieur de la première zone centrale (8) de sorte que le moyen conduit, en raison de sa souplesse, peut être dévié de façon articulée par rapport à la première zone centrale (8) sans que, dû à cela, des efforts importants soient transmis à la deuxième zone (10) en forme de bord.

13. Dispositif de raccordement selon l'une ou plusieurs des revendications précédentes, **caractérisé par le fait que** le corps de raccordement (4) porte de manière inamovible sur la face de la deuxième zone (10) en forme de bord, qui montre dans la direction opposée à la plaie ou sur celle (14) qui montre vers la plaie, un film de fixation (52) qui fait saillie radialement vers l'extérieur au-delà d'un bord périphérique (54) de la deuxième zone (10) en forme de bord d'au moins 5 mm, en particulier d'au moins 10 mm, et qui présente une couche d'adhésif sur sa face montrant vers la plaie, au moyen de laquelle il peut être appliqué de manière adhésive sur le pansement à pression négative.

14. Dispositif de raccordement selon la revendication 13, **caractérisé par le fait que** ledit film de fixation (52) comprend au moins une portion de film protecteur détachable sur la face montrant vers la plaie et au moins une portion de film support (58) sur la face montrant dans la direction opposée à la plaie, qui peut être retirée après que le dispositif de raccordement a été appliqué sur le pansement à pression négative.

15. Procédé de production d'un dispositif de raccordement selon l'une ou plusieurs des revendications précédentes 1 à 14, **caractérisé par le fait que** le corps de raccordement (4) avec ladite première zone centrale (8) et ladite deuxième zone (10) en forme de bord est fabriqué et fourni d'un seul tenant, et que sur la face de la deuxième zone (10) en forme de bord, montrant dans la direction opposée à la plaie, un film de fixation (52) qui est de préférence continu dans la direction circonférentielle (7) et présente un évidement central (60) est prévu en ce que le film de fixation (52) est planté par son évidement central (60) sur la première zone centrale (8), et que, ensuite, le film de fixation (52) adhère de manière non détachable, pour l'utilisation prévue, au moyen d'un adhésif sur la face (14) de la deuxième zone (10) en forme de bord, qui montre dans la direction opposée à la plaie, et fait saillie vers l'extérieur au-delà d'un bord périphérique (54) de la deuxième zone (10) en forme de bord, et que par la suite le moyen conduit (6) est introduit dans une ouverture (44) de la première zone centrale (8), qui débouche à peu près parallèlement au plan d'appui (12) et est fixé par liaison de matière et de manière étanche à la pression négative.

16. Système pour la thérapie par pression négative de plaies, comprenant un appareil comprenant un dispositif de génération de pression négative et ayant un raccord de pression négative et un raccord de rinçage ou d'instillation, **caractérisé par** un dispositif de raccordement (4) selon l'une ou plusieurs des revendications précédentes 1 à 14, dans lequel une lumière d'aspiration (40) du moyen conduit (6) est reliée fluidiquement au raccord de pression négative et la lumière d'alimentation (42) du moyen conduit (6) est reliée fluidiquement au raccord de rinçage ou d'instillation.
